# EUROPEAN PATENT SPECIFICATION

(11) **EP 2 367 292 B1**
(45) Date of publication and mention of the grant of the patent: **18.05.2016**
(21) Application number: 10194337.1
(22) Date of filing: 09.12.2010
(51) Int. Cl.: H04B 1/38

(54) **Watch type mobile terminal**
Uhrenartiges mobiles Endgerät
Terminal mobile de type montre

(30) Priority: 15.03.2010 KR 20100022981
(43) Date of publication of application: 21.09.2011
(73) Proprietor: LG ELECTRONICS INC., Yeongdeungpo-gu Seoul 07336 (KR)
(72) Inventor: Byun, Huiseob, Seoul, Rep. of Korea (KR); Won, Changbai, Gyeonggi-Do (KR); Yoo, Inseok, Seoul (KR)
(74) Representative: Ter Meer Steinmeister & Partner

(56) References cited:
- WO-A2-2005/103846
- US-B1- 6 825 751

## Description

### BACKGROUND OF THE INVENTION

### Field of the Invention

The present invention relates to a watch type mobile terminal including a window and that can be put on a user's wrist and a method for assembling the same.

### Description of the Related Art

A mobile terminal is a device that can be carried around and has one or more functions such as voice and video call communication, inputting and outputting information, storing data, and the like.

As such functions become more diversified, the watch type mobile terminal can support more complicated functions such as capturing images or video, reproducing music or video files, playing games, receiving broadcast signals, and the like. By comprehensively and collectively implementing such functions, the watch type mobile terminal may be embodied in the form of a multimedia player or device.

In order to implement various functions of such multimedia players or devices, the multimedia player requires sufficient support in terms of hardware or software, for which numerous attempts are being made and implemented. For example, a user interface allowing users to easily and conveniently search for and select one or more functions is provided.

Also, as mobile terminals are considered a personal mobile object that can express users' personality, various designs are required. Such demand on designs may include a watch type mobile terminal that can be put on users' wrist so as to be used.

As one of structural alterations and modifications allowing users to conveniently use the watch type mobile terminal, a window mounting structure extending a display area can be considered.

WO 2005/103846 A2 relates to an interactive watch, comprising first remote wireless transmission means and a communication module (E), connected to the watch body (1) by means of fixing means which may be easily disconnected and provided with second remote transmission means for the exchange of information with the first remote transmission means provided on the watch. Said communication module (E) further comprises a microphone/earphone interface and an elastic grip for fixing to the ear of the user.

US 6,825,751 B1 relates to a wristwatch comprising an ID Tag (data carrier) function applied to an RF-ID system. By these means, the wristwatch can be connected to a mobile terminal.

### SUMMARY OF THE INVENTION

Accordingly, one object of the present invention is to address the above-noted and other problems.

Another object of the present invention is to provide a watch type mobile terminal and a method for assembling the same that can be put on the user's wrist so as to be used and has a large display area.

Another object of the present invention is to provide a watch type mobile terminal having an improved waterproof structure and a method for assembling the same.

To achieve these and other advantages and in accordance with the purpose of the present invention, as embodied and broadly described herein, the present invention provides a watch type mobile terminal including: a main body having a window hole; a band connected with both sides of the main body; a support member having a central opening and having a first portion formed to surround the window hole and a second portion extending toward the central opening from the first portion in order to cover the edges of the window hole; a window having a front surface facing the exterior of the main body and a rear surface opposing the front surface formed on the opposite side of the front surface, and disposed such that at least a portion of the front surface is covered by the second portion of the supporting member at the window hole; and a ring member inserted between the window hole and the window to fix the window.

The object is also solved by a watch type mobile terminal comprising: a main body having an edge to define a window hole, the main body including a support member with a first portion to surround an outer perimeter of the window hole and a second portion that protrudes from the first portion toward a central area of the window hole and covers a portion of the edge of the window hole; a circuit board within the main body; a display on the circuit board; a band to couple to the main body; a window having a first surface and a second surface on an opposite side of the window, and the second portion of the support member covers a portion of the first surface of the window; and a ring member between the first portion of the support member and the window.

The support member may be mounted on one surface of the main body, and the first portion may be formed to cover one surface of the main body. The first portion may be protruded from the second portion. The ring member may be formed such that at least a portion thereof is mounted on the second portion. The first and second portions may be formed to have a step due to the protruded first portion. The step may be formed such that the hollow portion of the support member increases gradually in a direction from the front surface to the rear surface of the window.

In another embodiment of the present invention, an insertion portion is formed on an inner circumferential surface of the ring member to allow the side of the window to be inserted therein. A protrusion may be formed on an inner circumferential surface of the ring member such that it is protruded from the insertion portion toward the hollow portion in order to limit the insertion of the window. The ring member may be configured such that when the side of the window is inserted into the insertion portion, the ring member is elastically deformed between the window hole and the window.

In another embodiment of the present invention, the watch type mobile terminal may include a touch pad. The touch pad may be disposed to cover the rear surface of the window and configured to detect a touch input applied thereto. The touch pad may include an electrode film and a signal connection member. The electrode film may generate an electric signal corresponding to the touch input, and at least a portion of an outer circumference of the electrode film may be protruded in one direction. The signal connection member may be disposed to penetrate an open portion of the cover member. One end of the signal connection member may be connected to the protruded portion of the electrode film and the other end of the signal connection member may be connected to a circuit board to process the electric signal. The watch type mobile terminal may further comprise a support frame provided at the main body, the circuit board may be provided on the support frame.

The main body may include a first band connection portion at a first area where the band is coupled to the main body and a second band connection portion at a second area where the band is coupled to the main body. A protruding portion of the electrode film and the signal connection member may be provided at the first band connection portion. At the second band connection portion a hole to receive a camera window may be provided.

In another embodiment of the present invention, the signal connection member may be formed such that at least a portion thereof extends from an outer circumference of the electrode film and is wound toward the circuit board. The watch-type mobile terminal may include a display unit disposed to correspond to the hollow portion. The window, the display unit, and the circuit board may be laminated to be disposed, and the signal connection member may extend from the outer circumference of the electrode film and be connected to the circuit board through the display unit.

In another embodiment of the present invention, the watch-type mobile terminal may include a cover member. The cover member may be mounted on the main body and formed to cover edges of a rear surface of the window. The cover member may be configured as a hollow body, and at least a portion of a hollow portion of the cover member is open in a direction perpendicular to a central axis of the hollow portion. Thus, the cover member has a ring shape and comprises a central hole, wherein the ring shape or circumference of the cover member is open or interrupted. The window may have a circular shape, a touch pad is disposed on a rear surface of the window, the touch pad may include an electrode film formed to have a circular shape and having a portion of the circumference protruded toward an open portion of the cover member; and a signal connection member having one end connected with the protruded portion of the electrode film and the other end connected with the circuit board to process the electrical signal.

In another embodiment of the present invention, the watch type mobile terminal may include a gasket. The gasket may be disposed between one surface of the main body and the support member in order to limit an inflow of a fluid in the interior of the main body.

In another embodiment of the present invention, the support member may be formed to configure the case of the main body.

The object is also solved by a method for assembling a watch-type mobile terminal as described above, comprising the steps of: inserting the ring member into the support member from bottom-to-top (+y direction); inserting the window from bottom-to-top (+y direction) and fixing the window in the window hole by the ring member; and mounting the cover member on the rear surface of the front case to cover the ring member and the rear surface of the window.

Further scope of applicability of the present invention will become apparent from the detailed description given hereinafter. However, it should be understood that the detailed description and specific examples, while indicating preferred embodiments of the invention, are given by illustration only, since various changes and modifications within the spirit and scope of the invention will become apparent to those skilled in the art from this detailed description.

### BRIEF DESCRIPTION OF THE DRAWINGS

The present invention will become more fully understood from the detailed description given hereinbelow and the accompanying drawings, which are given by illustration only, and thus are not limitative of the present invention, and wherein:
FIG. 1 is a front perspective view of a watch type mobile terminal according to an exemplary embodiment of the present invention;
FIG. 2 is a rear perspective view of the watch type mobile terminal of FIG. 1;
FIG. 3 is an exploded view of the watch type mobile terminal of FIG. 1;
FIG. 4 is a sectional view of the watch type mobile terminal of FIG. 1 taken along line IV-IV;
FIG. 5 is a partial exploded view of a portion 'A' of the watch type mobile terminal in FIG. 4;
FIG. 6 is a sectional view of the watch type mobile terminal of FIG. 1 taken along line VI-VI;
FIGS. 7A to 7C are views showing the process of assembling the watch type mobile terminal of FIG. 3;
FIGS. 8 and 9 are sectional views showing another example of the watch type mobile terminal according to an exemplary embodiment of the present invention;
FIG. 10 is a schematic block diagram of the watch type mobile terminal according to an exemplary embodiment of the present invention; and
FIG. 11 is a sectional view of a watch type mobile terminal assembly according to the related art.

### DETAILED DESCRIPTION OF THE INVENTION

A watch type mobile terminal according to exemplary embodiments of the present invention will now be described in detail. In the following description, usage of suffixes such as 'module', 'part' or 'unit' used for referring to elements is given merely to facilitate explanation of the present invention, without having any significant meaning by itself.

In the present disclosure, the same or like reference numerals are used for the same or like elements, and a first explanation will be used. As used herein, the singular forms "a", "an" and "the" are intended to include the plural forms as well, unless the context clearly indicates otherwise.

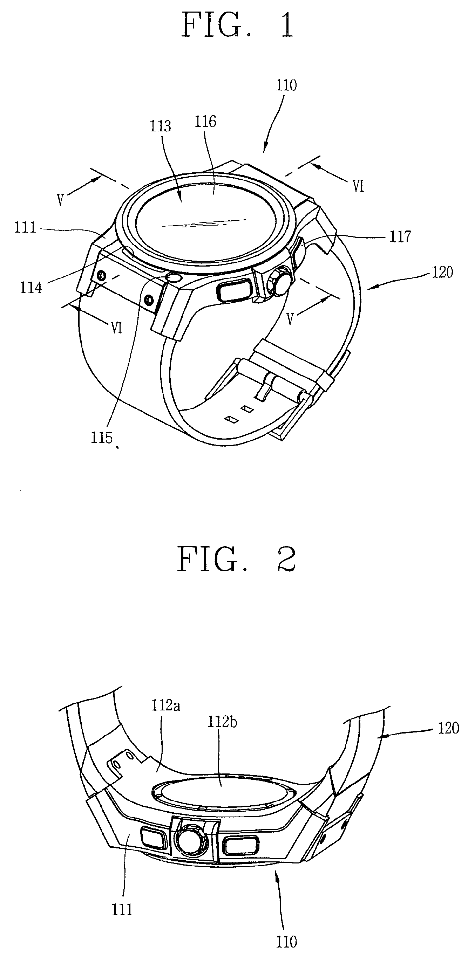

FIG. 1 is a front perspective view of a watch type mobile terminal according to an exemplary embodiment of the present invention.

A main body 110 of a watch type mobile terminal is connected to a band 120. The band 120 is connected to both sides of the main body 110.

The main body 110 is formed to include a wireless communication module 181 to transmit and receive radio signals to and from a mobile communication base station.

An outer appearance of the main body 110 is formed by a front case 111 and a rear case 112a (See FIG. 2). The front case 111 and the rear case 112a form an internal space in which various electronic components are installed.

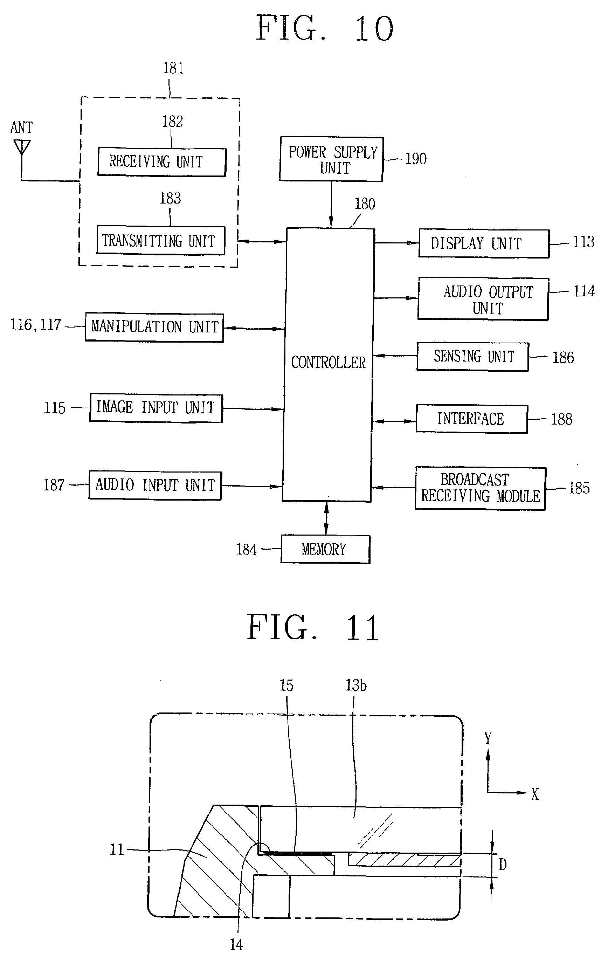

A display unit 113, an audio output unit 114, an image input unit 115, or user input units 116 and 117 (See FIG. 10) may be disposed on the terminal main body, mainly, on the front case 111.

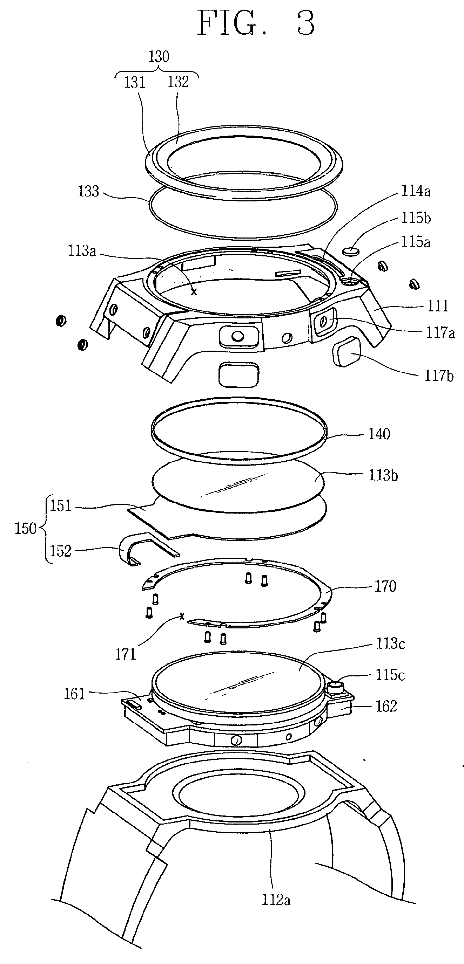

The display unit 113 occupies the most of circumferential surface of the front case 111. The display unit 113 may include a display 113c (See FIG. 3). The display 113c may include at least one of a Liquid Crystal Display (LCD), a Thin Film Transistor-LCD (TFT-LCD), an Organic Light Emitting Diode (OLED) display, a flexible display, a three-dimensional (3D) display, or the like. The display 113c may be configured to be transparent or light-transmissive to allow viewing of the exterior, which may be called a transparent display. A typical transparent display may be, for example, a TOLED (Transparent Organic Light Emitting Diode) display.

The watch type mobile terminal 100 may include two or more display units 113 according to its particular desired embodiment. For example, the display unit 113 may be formed to selectively display image information and time information according to a user information input.

The display unit 113 may be formed to include a touch sensor (or a touch pad) for receiving an input of information according to a user's touch. The touch sensor may be configured to detect the pressure of a touch input as well as a touch input position and area. The display unit 113 including the touch sensor forms a touch screen.

An audio output unit 114 and an image input unit 115 are disposed in an area adjacent to one of both ends of the display unit 113.

The audio output unit 114 may be implemented in the form of a speaker module for outputting a sound. The speaker module may include a receiver, a loud speaker, and the like.

The image input unit 115 may be implemented in the form of a camera module 115c (See FIG. 3) for capturing an image or vide of the user and so one.

An audio input unit 187 (See FIG. 10) may be disposed in an area adjacent to the other of the both end portions of the display unit 113. The audio input unit 187 may be implemented in the form of, for example, a microphone, in order to receive the user's voice, other sounds, and the like. Such an audio input unit 187 may be disposed on the side of the terminal main body.

The user input units 116 and 117 refer to units manipulated to receive a command for controlling the operation of the watch type mobile terminal. For example, the user input units 116 and 117 may be implemented as a dome switch or a touch screen which can receive information or commands input by a user in a pushing or touching manner, or implemented in a manner of using a wheel, a jog or a joystick to rotate keys.

The touch screen is used as the first user input unit 116 besides an output device. The second user input unit 117 may be disposed on the side of the front case 111.

Content inputted by the first and second user input units 116 and 117 may be set to be different. For example, the first user input unit 116 maybe formed to receive information such as numbers, characters, or symbols. The second user input unit 117 may be formed to receive START or END, or receive a command such as adjustment of the size of a sound outputted from the audio output unit 114 or changing into a touch recognition mode of the display unit 113, and the like.

FIG. 2 is a rear perspective view of the watch type mobile terminal of FIG. 1.

The rear case 112a is coupled to the front case 111 to confine the internal space. A cover 112b is mounted on the rear case 112a to cover the internal space.

The rear case 112a and the cover 112b may be formed by injection-molding a synthetic resin or may be made of a metallic material such as stainless steel (STS), titanium (Ti), etc.

An interface 188 (See FIG. 10) may be disposed on the side or the rear surface of the watch type mobile terminal.

The interface 188 may be at least one of a wired/wireless access terminal to be connected to an earphone, a short-range communication port (e.g., IrDA port, Bluetooth™ port, wireless LAN port, and the like), and a power supply terminal for supplying power to the mobile terminal. The interface 188 may be a card socket for accommodating an external card such as Subscriber Identification Module (SIM), User Identity Module (UIM), memory card for storing information, or the like.

For example, as the interface 188, a connection terminal (not shown) that can be connected to an external power supply device, may be formed at the side of the rear case 112a in order to supply power to at least one element of the mobile terminal. The connection terminal (not shown) may be electrically connected with a power supply unit 190 (See FIG. 10), e.g., a rechargeable battery, and the like, of the terminal.

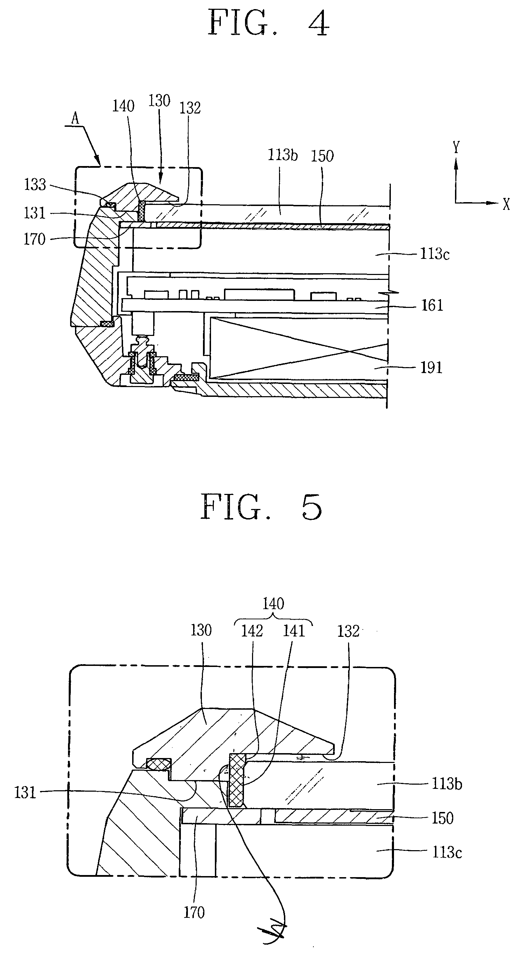

FIG. 3 is an exploded view of the watch type mobile terminal of FIG. 1, and FIG. 4 is a sectional view of the watch type mobile terminal of FIG. 1 taken along line IV-IV.

With reference to FIG. 3, a sound hole 114a and an image hole 115a are formed on the front surface of the front case 111, and a speaker module (not shown) and a camera module 115c are disposed at the inner side of the front case 111 such that the speaker module and the camera module correspond to the sound hole 114a and the image hole 115a. An image window 115b formed to be light-transmissive may be mounted on the image hole 115a. One surface of the front case 111 facing the exterior is defined as a front surface, and the other surface of the front case 111 facing an internal space is defined as a rear surface. The sound hole 114a and the speaker module form the audio output unit 114, and the image hole 115a and the camera module 115b form the image input unit 115 (See FIG. 1).

A keypad 117b to be press-manipulated by the user to receive a control command is mounted on the front case 111. In detail, at least a portion of the side of the front case 111 is recessed to form a keypad installation recess 117a, and the keypad 117b is mounted in the keypad installation recess 117a such that the keypad 117b is movable along the side of the keypad installation recess 117a.

A window hole 113a is formed on the front surface of the front case 111, and a support member 130 is mounted.

The support member 130 has a central opening and is made of a metal material. The support member 130 may be decorations of the watch type mobile terminal.

The support member 130 includes first and second areas 131 and 132.

The first area 131 is formed to laterally surround the window hole 113a, and the second area 132 extends to the central opening from the first area 131 to cover the edge of the window hole 113a. For example, the first area 131 has an annular shape and covers the front surface of the front case 111. The second area 132 is protruded from an inner circumference of the first area 131 toward the center and covers the edge of the window hole 113a.

A gasket 133 may be disposed between the front surface of the front case 111 and the support member 130. The gasket 133 seals a coupled part between the support member 130 and the front case 111 to prevent an introduction of a fluid into the interior of the terminal.

The window 113b may be disposed to correspond to the window hole 113a.

The window 113b may be made of a material allowing light to transmit therethrough, for example, a light-transmissive synthetic resin, tempered glass, or the like. The window 113b may include a portion through which light cannot pass. Such a portion may be made of a material not allowing light to transmit therethrough or surface-treated not to allow light to transmit therethrough.

In more detail, the window 113b may include a front surface facing the exterior of the terminal main body and a rear surface opposing the front surface. At least a portion of the front surface of the window 113b is disposed to be covered by the second area 132. Namely, one surface of the second area 132 faces the internal space of the terminal, and the front surface of the window 113b is disposed to face one surface of the second area 132.

With reference to the drawings, a ring member 140 is inserted between the window hole 113a and the window 113b. The window 113b and the window hole 113a may be formed to have a circular shape. The ring member 140 may be may fit between a face confining the window hole 113a and an outer circumferential surface of the window 113b in order to fix the window 113b.

A touch pad 150 may be mounted on the rear surface of the window 113b. The touch pad is disposed to cover the rear surface of the window 113b and formed to detect a touch input.

The touch pad 150 may be configured to convert a change in capacitance, potential gradient, and the like, generated at a particular portion of the display unit 113 in to an electrical input signal. However, the present invention is not limited thereto, and for example, the touch pad may be mounted on the front surface of the window 113b and detect pressure applied to a particular portion of the display unit 113.

With reference to the drawings, the touch pad 150 includes an electrode film 151 and a signal connection member 152.

The electrode film 151 generates an electrical signal corresponding to a touch input. The electrode film 151 is used as a means for calculating coordinates on a screen to detect a touch applied to the screen, and may be an indium tin oxide (ITO) electrode film, a carbon nanotube (CNT) electrode film, and the like. The electrode film 151 may have a circular or oval shape, and at least a portion of the outer circumference of the electrode film 151 may be protruded in one direction.

The signal connection member 152 may be, for example, a flexible printed circuit board (FPCB). One end of the signal connection member 152 may be connected with a protruded portion 151 a of the electrode film 151, and the other end may be connected with a circuit board 161 to process the electrical signal.

The circuit board 161 is mounted on a support frame 162, and the support frame 162 is disposed in a space confined by the front surface and the side surface of the front case 111.

At least one electronic component may be coupled to the support frame 162. The support frame 162 may be made of a material having a higher strength or rigidity than that of a material of the front case 111. For example, when the front case 111 is made of a synthetic resin, for example, polycarbonate, the support frame 162 may be made of stainless steel having a higher strength. Accordingly, the support frame 162 with a small thickness reinforces the strength of rigidity of the front case 111.

With reference to the drawings. The support frame 162 supports the display 113c at the front side and may be formed to allow a battery 191 (See FIG. 5) to be inserted on the rear surface.

The display 113c may have a circular or oval shape, and may be disposed to correspond to the hollow part of the window 113b and the support member 130. Accordingly, visual information outputted to the display 113c may be recognized from the exterior. The window 113b may have an area corresponding to the display 113c. The display 113c and the window 113b may configure the display unit 113 (See FIG. 1).

A cover member 170 may be mounted on the rear surface of the front case 111. The cover member 170 may be formed to cover the edges of the rear surface of the window 113b and may be formed as a hollow body. The cover member 170 serves to firmly fix the window 113b fit by the ring member 140. As illustrated, at least a portion of the hollow part of the cover member 170 is open in a direction perpendicular to a central axis of the hollow part.

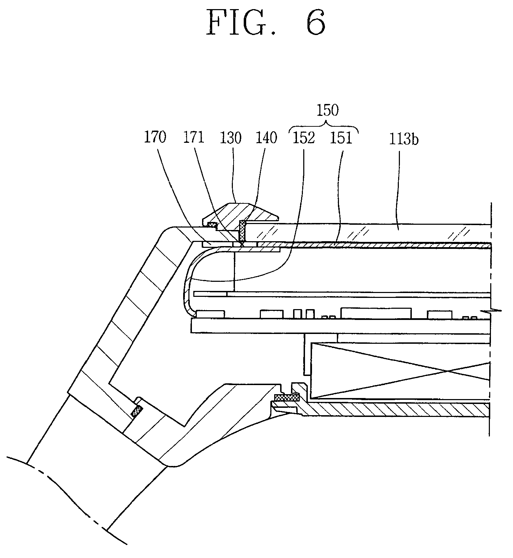

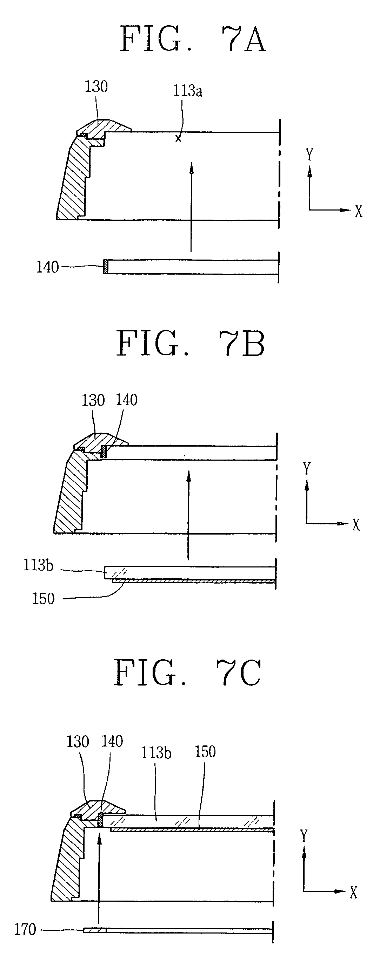

A mechanism for mounting the window will now be described in detail with reference to FIGS. 4 to 7C. FIG. 5 is a partial exploded view of a portion 'A' of the watch type mobile terminal in FIG. 4, FIG. 6 is a sectional view of the watch type mobile terminal of FIG. 1 taken along line VI-VI, and FIGS. 7A to 7C are views showing the process of assembling the watch type mobile terminal of FIG. 3.

With reference to FIG. 5, the first area 131 of the support member 130 is protruded from the second area 132. The first and second areas 131 and 132 form a step together as the first area 131 is protruded, and the step is formed such that the central opening of the support member 130 widens from the front surface of the window 113b toward the rear surface of the window 113b. Thus, when the window 113b is assembled, the window 113b can be inserted in a direction (+ Y direction) from the rear surface of the front case 111 to the front surface of the front case 111. In this manner, the support member 130 is formed not to generate an undercut in the + Y direction.

As illustrated, the ring member 140 is formed such that at least a portion is mounted on the second area 132, and the inserted window 113b is fixed by the ring member 140. The process of assembling the window 113b will now be described with reference to FIGS. 7A to 7C.

First, as shown in FIG. 7A, the ring member 140 is inserted into the second area 132 of the support member 130, and in this case, the ring member 140 is inserted in the +Y direction. Next, as shown in FIG. 7B, the window 113b is inserted in the + Y direction and fixed in the window hole 113a by the ring member 140. in this case, the window 113b is inserted in the + Y direction in a state that the touch pad 150 is mounted. Finally, the cover member 170 is mounted on the rear surface of the front case 111 to cover the ring member 140 and the rear surface of the window 113b.

The foregoing assembling process is a bottom-up type assembling process, which has an advantage in that a bezel of the window can be formed to be narrower, compared with a top-down type assembling process. The top-down type assembling process is applied to the structure for mounting a window 13b in the related art illustrated in FIG. 11.

FIG. 11 is a sectional view of a watch type mobile terminal assembly according to the related art. With reference to FIG. 11, a portion covering a window hole 13a from a front surface of a case 11 has a step toward an internal space. In detail, a step portion 14 is formed as a recess on the front surface of the case 11. The window 13b may be mounted on the step portion 14 by the medium of an adhesive 15 or a double-sided tape. At this time, an assembling is performed in a -Y direction. As illustrated, in order to allow the window 13 to be mounted on the step portion 14, the step portion 14 need to have a sufficient width and thickness, and thus, a window bezel is formed to be wider and thicker. The distance (D) between a display 13c and the window 13b is at least equal to or greater than the thickness of the step portion 14.

In comparison, with reference to FIG. 5, according to a window mounting mechanism, because the cover member is formed as a very thin plate, the distance between the display 113c and the window 113b can be smaller. Thus, a displayed screen image can be closer to the window and brightness of the screen can be improved.

With reference to FIG. 5, an insertion portion 141 is formed on the inner circumferential surface of the ring member 140 to allow the side of the window 113b to be inserted therein. In addition, a projection 142 may be formed on an inner circumferential surface of the ring member 140 and protruded toward the hollow part of the support member 130 in order to limit an insertion of the window 113b. For example, the section of the ring member 140 may extend in the +Y direction and bent toward the center of the ring member 140. Accordingly, the window 113b is inserted in the +Y direction along the insertion portion 141, and as the window 113b reaches the projection 142, it is fixed at a particular position within the window hole 113a.

When the side of the window 113b is inserted into the insertion portion 141, the ring member 140 is elastically deformed between the window hole 113a and the window 113b. In detail, the ring member 140 is made of an elastic material and pressurized to be compressed and deformed by the window 113b. Accordingly, the ring member 140 serve to fix the window 113b and seal it for waterproofing.

With reference to FIG. 6, the signal connection member 152 is disposed to extend from an outer circumference of the electrode film 151 such that at least a portion thereof is wound toward the circuit board 161. In more detail, the window 113b, the display 113c, and the circuit board 161 are disposed in a stacked manner, and the signal connection member 152 extends from the outer circumference of the electrode film 151, passing the display 113c, so as to be connected to the circuit board 161.

As illustrated, the terminal body has such an external appearance that at least a portion thereof is sloped in a direction in which it is wound on the user's wrist. The signal connection member 152 is wound at an internal space of the sloped portion.

As discussed above with reference to FIG. 3, at least a portion of the hollow part of the cover member 170 is open in a direction perpendicular to the central axis of the hollow part. Also, the electrode film 151 has a circular shape, and at least a portion of the outer circumference of the electrode film 151 is protruded toward the open portion 171 of the cover member 170. With reference to FIG. 6, the open portion 171 of the cover member 170 may be disposed to face the sloped portion of the terminal body.

The signal connection member 152 passes through the open portion 171 of the cover member 170. One end of the signal connection member 152 is connected to the protruded portion 151 a of the electrode film 151, and the other end of the signal connection member 152 is connected to the circuit board 161. If the window 13b is assembled in the top-down manner as illustrated in FIG. 11, it would be difficult to electrically connect the electrode film and the circuit board. However, in the exemplary embodiment of the present invention, the electrical connection can be implemented through the simple structure as described above.

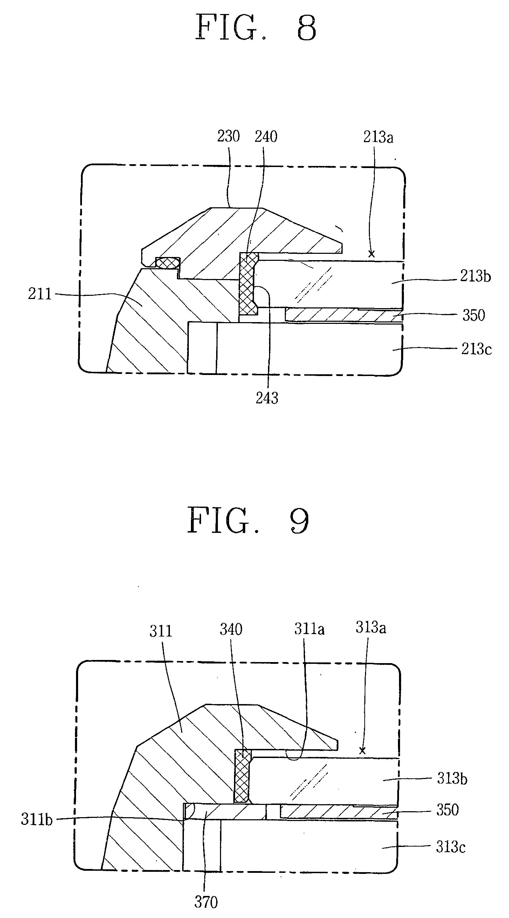

FIGS. 8 and 9 are sectional views showing other examples of the watch type mobile terminal according to exemplary embodiments of the present invention. Elements not described with reference to fig. 8 and 9 correspond to elements of the first embodiment as shown in figures 1-7.

With reference to FIG. 8, a recess portion 243 is formed on the inner circumferential surface of the ring member 240 to allow the side of the window 213b to be inserted therein. The recess portion 243 is formed to be perpendicular to the inner circumferential surface of the ring member 240, and the side of the window 213b is mounted on a bottom surface of the recess portion 243.

According to the structure, the window can be fit according to the elastic deformation of the ring member and can be fixed as the window is inserted in the recess portion 243. Thus, the window 113b can be firmly fixed even without the cover member 170 (See FIG. 5).

With reference to FIG. 9, the shape of the support member 330 can be implemented by a front case 311. Namely, the support member 330 forms one case of the terminal body. In detail, the front case 311 is formed to gradually widen toward the interior of a window hole 313a.

For example, the window hole 313a may form first and second step faces 311 a and 311b. The first and second step faces 311 a and 311 b are formed on a rear surface of the front case 311. The first step face 311 a may be a bezel of the window, and the second step face 311 b may allow the cover member 370 to be mounted thereon. With such a configuration, a window mounting mechanism assembled in the bottom-up manner can be implemented without using the support member 130 (See FIG. 5).

FIG. 10 is a schematic block diagram of the watch type mobile terminal according to an exemplary embodiment of the present invention; and

With reference to FIG. 10, the watch type mobile terminal according to the an exemplary embodiment of the present invention may include the display unit 113, the audio output unit 114, a video input unit 115, user input units 116 and 117, a power supply unit 190, a controller 180, a wireless communication module 181, a memory 184, a broadcast receiving module 185, a sensing unit 186, an audio input unit 187, and an interface 188.

The controller 180 may control an overall operation of the watch type mobile terminal. For example, the controller 190 may perform related control and processing for a voice call communication, data communication, telephony communication and the like.

The wireless communication module 181 may transmit and receive radio signals with a mobile communication base station via an antenna. For example, the wireless communication module 181 manages transmission and reception of audio data, text data, video data and control data under the control of the controller 180. To this end, the wireless communication module 181 may include a transmitting unit 162 for modulating and transmitting a signal to be sent, and a receiving unit 183 for demodulating a signal received.

The user input units 116 and 117 may be configured to provide the controller 180 with key input data input by a user to control the operations of the mobile terminal. The user input units 116 and 117 may include a dome switch, a touchpad (e.g., static pressure/capacitance), a jog wheel, a jog switch and the like.

The video input unit 115 processes image frames of still images or video obtained by an image sensor in a video call mode or a capturing mode. Such processed image frames are converted into image data displayable on the display unit 113 to be then output on the display unit 113.

The image frames processed by the video input unit 115 may be stored in the memory 184 under the control of the controller 180 or be sent to the exterior via the wireless communication module 181.

The audio input unit 187 receives an external audio signal via a microphone and processes it into electrical voice data, while the mobile terminal is in a particular mode, such as phone call mode, recording mode and voice recognition. The processed audio signal is converted into digital data.

Such processed digital data is converted into a data format transmittable to a mobile communication base station via the wireless communication module 181 when the mobile terminal is in the phone call mode, and then outputted to the wireless communication module 181. The processed voice data may be stored in the memory 184 when the mobile terminal is in a recording mode.

The audio input unit 187 may include assorted noise removing algorithms to remove noise generated in the course of receiving the external audio signal.

The display unit 113 displays information processed in the watch type mobile terminal. For example, when the watch type mobile terminal is in the phone call mode, the display unit 113 displays User Interface (UI) or (Graphic User Interface (GUI) related to the call under the control of the controller 180. When the watch type mobile terminal is in the video call mode or the image capturing mode, the display unit 113 displays a captured image or Ul or GUI under the control of the controller 180. When the display unit 113 includes a touch screen, it may be used as an input device as well as an output device.

The audio output unit 114 may convert audio data received from the wireless communication module 181 or audio data stored in the memory 184 and output the same to the exterior under the control of the controller 190 when the mobile terminal is in the call reception mode, a phone call mode, a recording mode, a voice recognition mode, or a broadcast receiving mode.

Also, the audio output unit 114 outputs an audio signal associated with a function (e.g., outputting a call receiving sound, a message receiving sound, or the like) performed in the watch type mobile terminal.

The sensing unit 186 detects a current status of the watch type mobile terminal. Namely, the sensing unit 186 detects an open/close status of the watch type mobile terminal, the location of the watch type mobile terminal, a presence or absence of user contact with the watch type mobile terminal and the like, and generates a sensing signal for controlling the operation of the watch type mobile terminal. For example, when the watch type mobile terminal is a slide type mobile phone, the sensing unit 186 senses the open or closed state of the slide type mobile terminal, and outputs the sensing results to the controller 180, so that the operation of the watch type mobile terminal can be controlled. Other examples include the sensing unit 186 sensing the presence or absence of power provided by the power supply 127, the presence or absence of a coupling or other connection between the interface 126 and an external device.

The interface 188 serves as an interface with external devices such as wired/wireless headsets, external chargers, wired/wireless data ports, card sockets (e.g., memory card, SIM/UIM card or the like) and the like, connected to the watch type mobile terminal. The interface 188 may allow the mobile terminal to receive data or power from external devices and transfer such data or power to each component inside the mobile terminal, or transmit internal data of the mobile terminal to external devices.

The memory 184 may store a program for the control and processing of the controller 190, or temporarily store input/output data (e.g., phone book data, messages, still images, video or the like).

Also, the memory 184 may store a program for controlling the operation of the watch type mobile terminal according to the present invention.

The memory 184 may include typically known hard disk, a card-type memory (e.g., SD or XD memory), a flash memory, RAM, ROM and the like.

The broadcast receiving module 185 may receive a broadcast signal transmitted via satellites or terrestrial waves and convert such broadcast signal into a broadcast data format capable of being output to the audio output unit 114 and the display unit 113 so as to output the converted signal to the controller 180. The broadcast receiving module 185 may also receive additional data associated with broadcasting (e.g., Electric Program Guide (EPG), channel list, or the like). The broadcast data converted in the broadcast receiving module 185 and the additional data may be stored in the memory 184.

The power supply 190 provides power required by the various components for the mobile terminal under the control of the controller 190. The provided power may be internal power, external power, or combinations thereof.

The watch type mobile terminal configured as described above according to an exemplary embodiment of the present invention has the following advantages. That is, because the front surface of the window is disposed to be covered by the support member and the ring member fixes the window, the width and thickness of the part where the window is mounted can be reduced, and accordingly, the display area can be increased.

Also, because the ring member which is elastically deformed fixes the window, a good waterproofing structure can be implemented.

In addition, because the front surface of the window is disposed on the inner surface of the support member, the window can be assembled in the direction from the inner surface toward the exterior. Accordingly, the distance between the window and the display can be reduced, a displayed screen image can become close on the window, and the brightness of the screen can be improved.

Moreover, the electrical connection between the touch pad and the circuit board can be facilitated through the touch pad mounted on the rear surface of the window.

As the exemplary embodiments may be implemented in several forms without departing from the characteristics thereof, it should also be understood that the above-described embodiments are not limited by any of the details of the foregoing description, unless otherwise specified, but rather should be construed broadly within its scope as defined in the appended claims. Therefore, various changes and modifications that fall within the scope of the claims, or equivalents of such scope are therefore intended to be embraced by the appended claims.

## Claims

1. A watch type mobile terminal comprising:
a main body (110) having a window hole (113a) defined by a ring shaped edge and a central area;
a circuit board (161) within the main body (110);
a display (113) on the circuit board (161);
a band (120) coupled to the main body (110);
**characterized by**
a ring shaped support member (130) having an outer portion (131) provided at the edge of the window hole (113a) and an inner portion (132) that extends from the outer portion (131) toward the central area of the window hole (113a) to cover the edge of the window hole;
a ring shaped window (113b) having a front surface facing an exterior of the main body (110) and a rear surface formed on an opposite side of the window (113b), and at least a portion of the front surface of the window (113b) is covered by the inner portion (132) of the support member (130); and
a ring member (140) provided between the outer portion (131) of the support member (130) and the window (113b).

2. A watch type mobile terminal comprising:
a main body (110) having a window hole (313 a) defined by a ring shaped edge and a central area,
a circuit board (161) within the main body (110);
a display (313c) on the circuit board (161);
a band (120) to couple to the main body (110);
**characterized by**
the main body (110) including a ring shaped support member (311, 330) with an outer portion (311b) to surround the edge of the window hole (313a) and an inner portion (311a) that protrudes from the outer portion (311b) toward the central area of the window hole (313a) and covers a portion of the edge of the window hole (313 a);
a window (313b) having a first surface and a second surface on an opposite side of the window (313b), and the inner portion of the support member (311, 330) covers a portion of the first surface of the window (313b); and
a ring member (340) provided between the outer portion of the support member (311, 330) and the window (313b).

3. The watch type mobile terminal of claim 1 or 2, further comprising a touch pad (150, 350) provided within the main body (110), the touch pad (150, 350) facing the rear surface of the window (113b, 313b).

4. The watch type mobile terminal of claim 3, wherein the touch pad (150, 350) includes an electrode film (151) having a protruding portion (151a) and a signal connection member (152) coupled to the protruding portion (151 a) of the electrode film (151).

5. The watch type mobile terminal of claim 4, wherein the signal connection member (152) is coupled to the circuit board (161) to electrically connect the electrode film (151) to the circuit board (161).

6. The watch type mobile terminal as claimed in any of the preceding claims, wherein the main body (110) includes a first band connection portion at a first area where the band (120) is coupled to the main body (110) and wherein the protruding portion (151a) of the electrode film (151) and the signal connection member (152) are provided at the first band connection portion.

7. The watch type mobile terminal as claimed in any of claims 4-6, wherein a portion of the signal connection member (152) extends from an outer circumference of the electrode film (151) and is bent toward the circuit board (161).

8. The watch type mobile terminal as claimed in any of the preceding claims, wherein the inner portion (132, 311 a) of the support member (130, 311, 330) protrudes from the outer portion (131, 311b) of the support member (130, 311, 330).

9. The watch type mobile terminal of claim 8, wherein the outer portion (131, 311b) and the inner portion (132, 311a) of the support member (130, 311, 330) have a step based on a protruded portion, and the step is formed such that a hollow portion (113a, 313a) of the support member (130, 311, 330) increases gradually in a direction from the first surface of the window (113b, 313b) to the second surface of the window (113b, 313b).

10. The watch type mobile terminal of claim 1, wherein the ring member (240) includes an recess portion (243) formed on an inner circumferential surface of the ring member (240), and the window (213b) is provided into the recess portion (243).

11. The watch type mobile terminal of claim 10, wherein the ring member (240) further includes a protrusion formed on the inner circumferential surface such that the protrusion protrudes from the recess portion (243) toward a hollow portion (213 a) of the support member (230).

12. The watch type mobile terminal according to any of the preceding claims, wherein the ring member (140, 240, 340) elastically deforms when the window (113b, 213b, 313b) is inserted into the ring member (140, 240, 340).

13. The watch type mobile terminal according to any of claims 1-9, further comprising:
a cover member (170, 370) on the main body (110) to cover an edge of the rear surface of the window (113b, 313b).

14. The watch type mobile terminal of claim 1, further comprising:
a gasket (133) between one surface of the main body (110) and the support member (130, 230) in order to limit inflow of fluid into an interior of the main body (110).

15. A method for assembling a watch-type mobile terminal according to any one of claims 1-9, comprising the steps of:
inserting the ring member (140, 340) into the support member (130, 330) from bottom-to-top (+y direction);
inserting the window (113b, 313b) from bottom-to-top (+y direction) and fix the window (113b, 313b) in the window hole (113a, 313a) by the ring member (140, 340); and
mounting a cover member (170, 370) on the rear surface of the front case (111, 311) to cover the ring member (140, 340) and the rear surface of the window (113b, 313b).

## Patentansprüche

1. Uhrenartiges mobiles Endgerät, das Folgendes umfasst:
einen Hauptkörper (110), der eine Fensteröffnung (113a) aufweist, die durch einen ringförmigen Rand und einen Mittelbereich definiert ist;
eine Leiterplatte (161) innerhalb des Hauptkörpers (110);
eine Anzeigevorrichtung (113) auf der Leiterplatte (161);
ein Band (120), das an den Hauptkörper gekoppelt ist (110);
**gekennzeichnet durch**
ein ringförmiges Tragelement (130), das einen äußeren Abschnitt (131), der an dem Rand der Fensteröffnung (113a) vorgesehen ist, und einen inneren Abschnitt (132) auf weist, der sich von dem äußeren Abschnitt (131) in Richtung des Mittelbereichs der Fensteröffnung (113a) erstreckt, um den Rand der Fensteröffnung abzudecken;
ein ringförmiges Fenster (113b), das eine Vorderfläche, die zu einer Außenseite des Hauptkörpers (110) zeigt, und eine Rückfläche aufweist, die auf der gegenüberliegenden Seite des Fensters (113b) gebildet ist, und wobei mindestens ein Abschnitt der Vorderfläche des Fensters (113b) von dem inneren Abschnitt (132) des Tragelements (130) bedeckt ist; und
ein Ringelement (140), das zwischen dem äußeren Abschnitt (131) des Tragelements (130) und dem Fenster (113b) vorgesehen ist.

2. Uhrenartiges mobiles Endgerät, das Folgendes umfasst:
einen Hauptkörper (110), der eine Fensteröffnung (313a) aufweist, die durch einen ringförmigen Rand und einen Mittelbereich definiert ist;
eine Leiterplatte (161) innerhalb des Hauptkörpers (110);
eine Anzeigevorrichtung (313c) auf der Leiterplatte (161);
ein Band (120), das an den Hauptkörper gekoppelt ist (110);
**gekennzeichnet durch**
den Hauptkörper (110), der ein ringförmiges Tragelement (311, 330) mit einem äuβeren Abschnitt (311b), um den Rand der Fensteröffnung (313a) zu umgeben, und einen inneren Abschnitt (311a) enthält, der aus dem äußeren Abschnitt (311b) in Richtung des Mittelbereichs der Fensteröffnung (313a) vorsteht und einen Abschnitt des Randes der Fensteröffnung (313a) bedeckt;
ein Fenster (313b), das eine erste Oberfläche und auf einer gegenüberliegenden Seite des Fensters (313b) eine zweite Oberfläche aufweist, und wobei der innere Abschnitt des Tragelements (311, 330) einen Abschnitt der ersten Oberfläche des Fensters (313b) bedeckt; und
ein Ringelement (340), das zwischen dem äußeren Abschnitt des Tragelements (311, 330) und dem Fenster (313b) vorgesehen ist.

3. Uhrenartiges mobiles Endgerät nach Anspruch 1 oder 2, das ferner ein Touchpad (150, 350) umfasst, das innerhalb des Hauptkörpers (110) vorgesehen ist, wobei das Touchpad (150, 350) zu der Rückfläche des Fensters (113b, 313b) zeigt.

4. Uhrenartiges mobiles Endgerät nach Anspruch 3, wobei das Touchpad (150, 350) eine Elektrodenschicht (151), die einen vorstehenden Abschnitt (151a) aufweist, und ein Signalverbindungselement (152) enthält, das an den vorstehenden Abschnitt (151a) der Elektrodenschicht (151) gekoppelt ist.

5. Uhrenartiges mobiles Endgerät nach Anspruch 4, wobei das Signalverbindungselement (152) an die Leiterplatte (161) gekoppelt ist, um die Elektrodenschicht (151) mit der Leiterplatte (161) elektrisch zu verbinden.

6. Uhrenartiges mobiles Endgerät nach einem der vorhergehenden Ansprüche, wobei der Hauptkörper (110) in einem ersten Bereich einen ersten Bandverbindungsabschnitt enthält, an dem das Band (120) an den Hauptkörper (110) gekoppelt ist, und wobei der vorstehende Abschnitt (151a) der Elektrodenschicht (151) und das Signalverbindungselement (152) in dem ersten Bandverbindungsabschnitt vorgesehen sind.

7. Uhrenartiges mobiles Endgerät nach einem der Ansprüche 4-6, wobei sich ein Abschnitt des Signalverbindungselements (152) aus einem äußeren Umfang der Elektrodenschicht (151) erstreckt und in Richtung der Leiterplatte (161) gebogen ist.

8. Uhrenartiges mobiles Endgerät nach einem der vorhergehenden Ansprüche, wobei der innere Abschnitt (132, 311a) des Tragelements (130, 311, 330) aus dem äußeren Abschnitt (131, 311b) des Tragelements (130, 311, 330) vorsteht.

9. Uhrenartiges mobiles Endgerät nach Anspruch 8, wobei der äußeren Abschnitt (131, 311b) und der innere Abschnitt (132, 311a) des Tragelements (130, 311, 330) eine Stufe aufweisen, die auf einem vorstehenden Abschnitt basiert, und wobei die Stufe derart gebildet ist, dass ein hohler Abschnitt (113a, 313a) des Tragelements (130, 311, 330) in einer Richtung von der ersten Oberfläche des Fensters (113b, 313b) zu der zweiten Oberfläche des Fensters (113b, 313b) allmählich größer wird.

10. Uhrenartiges mobiles Endgerät nach Anspruch 1, wobei das Ringelement (240) einen Aussparungsabschnitt (243) enthält, der in einer inneren Umfangsfläche des Ringelements (240) gebildet ist, und wobei das Fenster (213b) in dem Aussparungsabschnitt (243) vorgesehen ist.

11. Uhrenartiges mobiles Endgerät nach Anspruch 10, wobei das Ringelement (240) ferner einen Vorsprung enthält, der auf der inneren Umfangsfläche gebildet ist, so dass der Vorsprung in Richtung eines hohlen Abschnitts (213a) des Tragelements (230) aus dem Aussparungsabschnitt (243) vorsteht.

12. Uhrenartiges mobiles Endgerät nach einem der vorhergehenden Ansprüche, wobei sich das Ringelement (140, 240, 340) elastisch verformt, wenn das Fenster (113b, 213b, 313b) in das Ringelement (140, 240, 340) eingesetzt wird.

13. Uhrenartiges mobiles Endgerät nach einem der Ansprüche 1-9, das ferner Folgendes umfasst:
ein Abdeckelement (170, 370) auf dem Hauptkörper (110), um einen Rand der Rückfläche des Fensters (113b, 313b) zu bedecken.

14. Uhrenartiges mobiles Endgerät nach Anspruch 1, das ferner Folgendes umfasst:
einen Dichtring (133) zwischen einer Oberfläche des Hauptkörpers (110) und dem Tragelement (130, 230), um den Eintritt eines Fluids in eine Innenseite des Hauptkörpers (110) einzuschränken.

15. Verfahren zum Zusammenbauen eines uhrenartigen mobilen Endgeräts nach einem der Ansprüche 1-9, das die folgenden Schritte umfasst:
Einsetzen des Ringelements (140, 340) in das Tragelement (130, 330) von unten nach oben (+y-Richtung);
Einsetzen des Fensters (113b, 313b) in das Tragelement (130, 330) von unten nach oben (+y-Richtung) und Befestigen des Fensters (113b, 313b) in der Fensteröffnung (113a, 313a) durch das Ringelement (140, 340); und
Montieren eines Abdeckelements (170, 370) auf der Rückfläche des vorderen Gehäuses (111, 311), um das Ringelement (140, 340) und die Rückfläche des Fensters (113b, 313b) abzudecken.

## Revendications

1. Terminal mobile de type montre, comprenant :
un corps principal (110) comportant un trou à fenêtre (113a) défini par un bord de forme annulaire et une zone centrale ;
un circuit imprimé (161) dans le corps principal (110) ;
un afficheur (113) sur le circuit imprimé (161) ;
une bande (120) couplée au corps principal (110) ;
**caractérisé par**
un élément de forme annulaire (130) comportant une section extérieure (131) prévue sur le bord du trou à fenêtre (113a) et une section intérieure (132) qui s'étend de la section extérieure (131) vers la zone centrale du trou à fenêtre (113a) pour couvrir le bord du trou à fenêtre ;
une fenêtre de forme annulaire (113b) comportant une surface avant tournée vers un extérieur du corps principal (110) et une surface arrière formée sur une face opposée de la fenêtre (113b), et en ce qu'au moins une section de la surface avant de la fenêtre (113b) est couverte par la section intérieure (132) de l'élément support (130) ; et
un élément annulaire (140) prévu entre la section extérieure (131) de l'élément support (130) et la fenêtre (113b).

2. Terminal mobile de type montre comprenant :
un corps principal (110) comportant un trou à fenêtre (313a) défini par un bord de forme annulaire et une zone centrale,
un circuit imprimé (161) dans le corps principal (110) ;
un afficheur (313c) sur le circuit imprimé (161) ;
une bande (120) couplée au corps principal (110) ;
**caractérisé par**
**le fait que** le corps principal (110) inclut un élément support de forme annulaire (311, 330) doté d'une section extérieure (311b) pour entourer le bord du trou à fenêtre (313a) et une section intérieure (311a) qui dépasse de la section extérieure (311b) vers la zone centrale du trou à fenêtre (313a) et couvre une section du bord du trou à fenêtre (313a) ;
une fenêtre (313b) ayant une première surface et une seconde surface sur une face opposée de la fenêtre (313b), et en ce que la section intérieure de l'élément support (311, 330) couvre une section de la première surface de la fenêtre (313b) ; et
un élément annulaire (340) prévu entre la section extérieure de l'élément support (311, 330) et la fenêtre (313b).

3. Terminal mobile de type montre selon la revendication 1 ou 2, comprenant en outre un pavé tactile (150, 350) prévu dans le corps principal (110), le pavé tactile (150, 350) étant tourné vers la surface arrière de la fenêtre (113b, 313b).

4. Terminal mobile de type montre selon la revendication 3, dans lequel le pavé tactile (150, 350) comprend un film à électrode (151) doté d'une section proéminente (151a) et un élément de connexion de signal (152) couplé à la section proéminente (151a) du film à électrode (151).

5. Terminal mobile de type montre selon la revendication 4, dans lequel l'élément de connexion de signal (152) est couplé au circuit imprimé (161) pour connecter électriquement le film à électrode (151) au circuit imprimé (161).

6. Terminal mobile de type montre selon l'une quelconque des revendications précédentes, dans lequel le corps principal (110) comprend une première section de connexion de bande dans une première zone où la bande (120) est couplée au corps principal (110) et dans lequel la section proéminente (151a) du film à électrode (151) et l'élément de connexion de signal (152) sont prévus dans la première section de connexion de bande.

7. Terminal mobile de type montre selon l'une quelconque des revendications 4-6, dans lequel une section de l'élément de connexion de signal (152) s'étend depuis une circonférence extérieure du film à électrode (151) et est fléchie vers le circuit imprimé (161).

8. Terminal mobile de type montre selon l'une quelconque des revendications précédentes, dans lequel la section intérieure (132, 311a) de l'élément support (130, 311, 330) dépasse de la section extérieure (131, 311b) de l'élément support (130, 311, 330).

9. Terminal mobile de type montre selon la revendication 8, dans lequel la section extérieure (131, 311b) et la section intérieure (132, 311a) de l'élément support (130, 311, 330) ont une marche basée sur une section proéminente et la marche est formée de manière à ce qu'une section creuse (113a, 313a) de l'élément support (130, 311, 330) augmente graduellement dans un sens allant de la première surface de la fenêtre (113b, 313b) vers la seconde surface de la fenêtre (113b, 313b).

10. Terminal mobile de type montre selon la revendication 1, dans lequel l'élément annulaire (240) comprend une section en retrait (243) formée sur une surface circonférentielle intérieure de l'élément annulaire (240), et la fenêtre (213b) est prévue dans la section en retrait (243).

11. Terminal mobile de type montre selon la revendication 10, dans lequel l'élément annulaire (240) comprend en outre une saillie formée sur la surface circonférentielle intérieure de manière à ce que la saillie dépasse de la section en retrait (243) vers une section creuse (213a) de l'élément support (230).

12. Terminal mobile de type montre selon l'une quelconque des revendications précédentes, dans lequel l'élément annulaire (140, 240, 340) se déforme élastiquement lorsque la fenêtre (113b, 213b, 313b) est insérée dans l'élément annulaire (140, 240, 340).

13. Terminal mobile de type montre selon l'une quelconque des revendications 1-9, comprenant en outre :
un élément de recouvrement (170, 370) sur le corps principal (110) pour couvrir un bord de la surface arrière de la fenêtre (113b, 313b).

14. Terminal mobile de type montre selon la revendication 1, comprenant en outre :
un joint (133) entre une surface du corps principal (110) et l'élément support (130, 230) afin de limiter l'afflux de fluide dans un intérieur du corps principal (110).

15. Procédé d'assemblage de d'une terminale mobile de type montre selon l'une quelconque des revendications 1-9, comprenant les étampes :
d'insertion de l'élément annulaire (140, 340) dans l'élément support (130, 330) du bas vers le haut (sens +y) ;
d'insertion de la fenêtre (113b, 313b) du bas vers le haut (sens +y) et de fixation de la fenêtre (113b, 313b) dans le trou à fenêtre (113a, 313a) par l'élément annulaire (140, 340) ; et
de montage d'un élément de recouvrement (170, 370) sur la face arrière du compartiment avant (111, 311) afin de couvrir l'élément annulaire (140, 340) et la surface arrière de la fenêtre (113b, 313b).
